(19) Europäisches Patentamt
European Patent Office
Office européen des brevets

(11) **EP 2 631 637 A2**

(12) **EUROPEAN PATENT APPLICATION**

(43) Date of publication:
**28.08.2013 Bulletin 2013/35**

(51) Int Cl.:
*G01N 27/22* (2006.01)　*G01N 27/72* (2006.01)
*G01N 33/40* (2006.01)

(21) Application number: **13156139.1**

(22) Date of filing: **21.02.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.02.2012 US 201213401335**

(71) Applicant: **Varel International, Ind., L.P.**
**Carrollton, TX 75006 (US)**

(72) Inventors:
• **King, William W.**
**Houston, TX Texas 77069 (US)**
• **Bellin, Federico**
**The Woodlands, TX Texas 77381 (US)**
• **Chintamaneni, Vamsee**
**Houston, TX Texas 77064 (US)**

(74) Representative: **Smee, Anthony James Michael**
**Gill Jennings & Every LLP**
**The Broadgate Tower**
**20 Primrose Street**
**London EC2A 2ES (GB)**

(54) **Use of capacitance and eddy currents to analyze polycrystalline diamond**

(57)　A method for non-destructively characterizing one or more regions within an ultra-hard polycrystalline structure, such as a polycrystalline diamond compact, using capacitance and eddy current measurements. The eddy current measurements include at least one of an impedance amplitude and a phase shift angle. The capacitance is measured one or more times and compared to a first calibration curve to determine an estimated leaching depth within the polycrystalline structure. A first data scattering range is ascertained from the capacitance measurements to determine a relative porosity of the polycrystalline structure or the leaching quality within the polycrystalline structure. The eddy current is measured one or more times and compared to a second calibration curve to determine an estimated leaching depth within the polycrystalline structure. A second data scattering range is ascertained from the eddy current measurements to determine a relative porosity of the polycrystalline structure or the leaching quality within the polycrystalline structure. Results from both measurements are used to ascertain an overall quality of the polycrystalline structure.

FIG. 4

EP 2 631 637 A2

## Description

## RELATED APPLICATIONS

The present application is related to U.S. Patent Application No. 13/401,188, entitled "Use of Capacitance to Analyze Polycrystalline Diamond" and filed on February 21, 2012, U.S. Patent Application No. 13/401,231, entitled "Use of Eddy Currents to Analyze Polycrystalline Diamond" and filed on February 21, 2012, and U.S. Patent Application No. 13/401,452, entitled "Method To Improve The Performance Of A Leached Cutter" and filed on February 21, 2012, which are all incorporated by reference herein.

## TECHNICAL FIELD

The present invention relates generally to methods and apparatuses for measuring characteristics of one or more regions within an ultra-hard polycrystalline structure; and more particularly, to non-destructive methods and apparatuses for measuring the leaching depth within the ultra-hard polycrystalline structure and/or characterizing at least a portion of the ultra-hard polycrystalline structure, such as the ones used in forming polycrystalline diamond compact ("PDC") cutters, using at least capacitance and eddy current measurements.

## BACKGROUND

Polycrystalline diamond compacts ("PDC") have been used in industrial applications, including rock drilling applications and metal machining applications. Such compacts have demonstrated advantages over some other types of cutting elements, such as better wear resistance and impact resistance. The PDC can be formed by sintering individual diamond particles together under the high pressure and high temperature ("HPHT") conditions referred to as the "diamond stable region," which is typically above forty kilobars and between 1,200 degrees Celsius and 2,000 degrees Celsius, in the presence of a catalyst/solvent which promotes diamond-diamond bonding. Some examples of catalyst/solvents for sintered diamond compacts are cobalt, nickel, iron, and other Group VIII metals. PDCs usually have a diamond content greater than seventy percent by volume, with about eighty percent to about ninety-eight percent being typical. An unbacked PDC can be mechanically bonded to a tool (not shown), according to one example. Alternatively, the PDC is bonded to a substrate, thereby forming a PDC cutter, which is typically insertable within a downhole tool (not shown), such as a drill bit or a reamer.

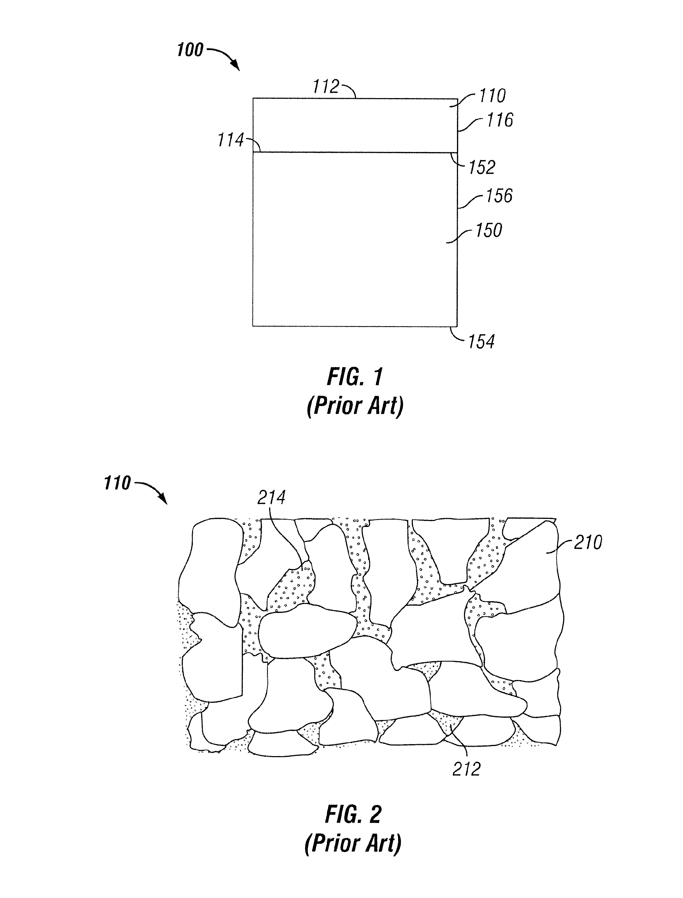

Figure 1 shows a side view of a PDC cutter 100 having a polycrystalline diamond ("PCD") cutting table 110, or compact, in accordance with the prior art. Although a PCD cutting table 110 is described in the exemplary embodiment, other types of cutting tables, including polycrystalline boron nitride ("PCBN") compacts, are used in alternative types of cutters. Referring to Figure 1, the PDC cutter 100 typically includes the PCD cutting table 110 and a substrate 150 that is coupled to the PCD cutting table 110. The PCD cutting table 110 is about one hundred thousandths of an inch (2.5 millimeters) thick; however, the thickness is variable depending upon the application in which the PCD cutting table 110 is to be used.

The substrate 150 includes a top surface 152, a bottom surface 154, and a substrate outer wall 156 that extends from the circumference of the top surface 152 to the circumference of the bottom surface 154. The PCD cutting table 110 includes a cutting surface 112, an opposing surface 114, and a PCD cutting table outer wall 116 that extends from the circumference of the cutting surface 112 to the circumference of the opposing surface 114. The opposing surface 114 of the PCD cutting table 110 is coupled to the top surface 152 of the substrate 150. Typically, the PCD cutting table 110 is coupled to the substrate 150 using a high pressure and high temperature ("HPHT") press. However, other methods known to people having ordinary skill in the art can be used to couple the PCD cutting table 110 to the substrate 150. In one embodiment, upon coupling the PCD cutting table 110 to the substrate 150, the cutting surface 112 of the PCD cutting table 110 is substantially parallel to the substrate's bottom surface 154. Additionally, the PDC cutter 100 has been illustrated as having a right circular cylindrical shape; however, the PDC cutter 100 is shaped into other geometric or non-geometric shapes in other exemplary embodiments. In certain exemplary embodiments, the opposing surface 114 and the top surface 152 are substantially planar; however, the opposing surface 114 and the top surface 152 are non-planar in other exemplary embodiments. Additionally, according to some exemplary embodiments, a bevel (not shown) is formed around at least the circumference of the cutting surface 112.

According to one example, the PDC cutter 100 is formed by independently forming the PCD cutting table 110 and the substrate 150, and thereafter bonding the PCD cutting table 110 to the substrate 150. Alternatively, the substrate 150 is initially formed and the PCD cutting table 110 is subsequently formed on the top surface 152 of the substrate 150 by placing polycrystalline diamond powder onto the top surface 152 and subjecting the polycrystalline diamond powder and the substrate 150 to a high temperature and high pressure process. Alternatively, the substrate 150 and the PCD cutting table 110 are formed and bonded together at about the same time. Although a few methods of forming the PDC cutter 100 have been briefly mentioned, other methods known to people having ordinary skill in the art can be used.

According to one example for forming the PDC cutter 100, the PCD cutting table 110 is formed and bonded to the substrate 150 by subjecting a layer of diamond powder and a mixture of tungsten carbide and cobalt powders to HPHT conditions. The cobalt is typically mixed with tungsten carbide and positioned where the substrate 150 is to be formed. The diamond powder is placed on top of the cobalt and tungsten carbide mixture and positioned where the PCD cutting table 110 is to be formed. The entire powder mixture is then subjected to HPHT conditions so that the cobalt melts and facilitates the cementing, or binding, of the tungsten carbide to form the substrate 150. The melted cobalt also diffuses, or infiltrates, into the diamond powder and acts as a catalyst for synthesizing diamond bonds and forming the PCD cutting table 110. Thus, the cobalt acts as both a binder for cementing the tungsten carbide and as a catalyst/ solvent for sintering the diamond powder to form diamond-diamond bonds. The cobalt also facilitates in forming strong bonds between the PCD cutting table 110 and the cemented tungsten carbide substrate 150.

Cobalt has been a preferred constituent of the PDC manufacturing process. Traditional PDC manufacturing processes use cobalt as the binder material for forming the substrate 150 and also as the catalyst material for diamond synthesis because of the large body of knowledge related to using cobalt in these processes. The synergy between the large bodies of knowledge and the needs of the process have led to using cobalt as both the binder material and the catalyst material. However, as is known in the art, alternative metals, such as iron, nickel, chromium, manganese, and tantalum, and other suitable materials, can be used as a catalyst for diamond synthesis. When using these alternative materials as a catalyst for diamond synthesis to form the PCD cutting table 110, cobalt, or some other material such as nickel chrome or iron, is typically used as the binder material for cementing the tungsten carbide to form the substrate 150. Although some materials, such as tungsten carbide and cobalt, have been provided as examples, other materials known to people having ordinary skill in the art can be used to form the substrate 150, the PCD cutting table 110, and the bonds between the substrate 150 and the PCD cutting table 110.

Figure 2 is a schematic microstructural view of the PCD cutting table 110 of Figure 1 in accordance with the prior art. Referring to Figures 1 and 2, the PCD cutting table 110 has diamond particles 210 bonded to other diamond particles 210, one or more interstitial spaces 212 formed between the diamond particles 210, and cobalt 214, or some other catalyst, deposited within one or more of the interstitial spaces 212. During the sintering process, the interstitial spaces 212, or voids, are formed between the carbon-carbon bonds and are located between the diamond particles 210. The diffusion of cobalt 214 into the diamond powder results in cobalt 214 being deposited within these interstitial spaces 212 that are formed within the PCD cutting table 110 during the sintering process.

Once the PCD cutting table 110 is formed and placed into operation, the PCD cutting table 110 is known to wear quickly when the temperature reaches a critical temperature. This critical temperature is about 750 degrees Celsius and is reached when the PCD cutting table 110 is cutting rock formations or other known materials. The high rate of wear is believed to be caused by the differences in the thermal expansion rate between the diamond particles 210 and the cobalt 214 and also by the chemical reaction, or graphitization, that occurs between cobalt 214 and the diamond particles 210. The coefficient of thermal expansion for the diamond particles 210 is about $1.0 \times 10^{-6}$ millimeters$^{-1}$ x Kelvin$^{-1}$ ("mm$^{-1}$K$^{-1}$"), while the coefficient of thermal expansion for the cobalt 214 is about $13.0 \times 10^{-6}$ mm$^{-1}$K$^{-1}$. Thus, the cobalt 214 expands much faster than the diamond particles 210 at temperatures above this critical temperature, thereby making the bonds between the diamond particles 210 unstable. The PCD cutting table 110 becomes thermally degraded at temperatures above about 750 degrees Celsius and its cutting efficiency deteriorates significantly.

Efforts have been made to slow the wear of the PCD cutting table 110 at these high temperatures. These efforts include performing a leaching process on the PCD cutting table 110, which removes some of the cobalt 214 from the interstitial spaces 212. These leaching processes, which includes, but is not limited to, an acid leaching process and/or an electrolytic leaching process, is known to persons having ordinary skill in the art and is not described herein for the sake of brevity. By removing some of the cobalt 214, or catalyst, from the PCD cutting table 110, the thermal degradation of the PCD structure is reduced.

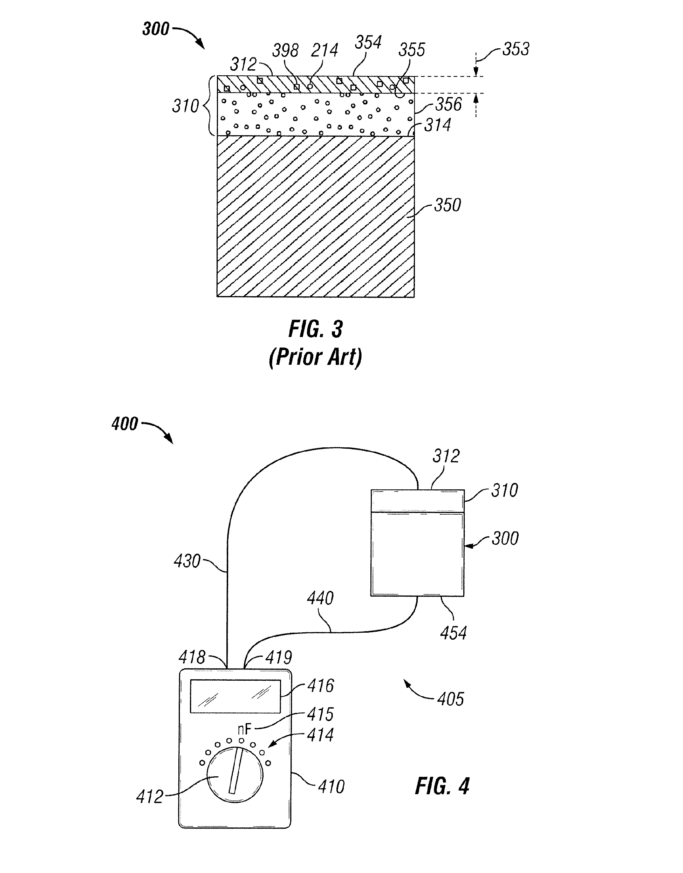

Figure 3 shows a cross-section view of a leached PDC cutter 300 having a PCD cutting table 310 that has been at least partially leached in accordance with the prior art. Referring to Figure 3, the PDC cutter 300 includes the PCD cutting table 310 coupled to a substrate 350. The substrate 350 is similar to substrate 150 (Figure 1) and is not described again for the sake of brevity. The PCD cutting table 310 is similar to the PCD cutting table 110 (Figure 1), but includes a leached layer 354 and an unleached layer 356. The leached layer 354 extends from the cutting surface 312, which is similar to the cutting surface 112 (Figure 1), towards an opposing surface 314, which is similar to the opposing surface 114 (Figure 1). In the leached layer 354, at least a portion of the cobalt 214 has been removed from within the interstitial spaces 212 (Figure 2) using at least one leaching process mentioned above. Thus, the leached layer 354 has been leached to a desired depth 353. However, during the leaching process, one or more by-product materials 398 are formed and deposited within some of the interstitial spaces 212 (Figure 2) in the leached layer 354. The unleached layer 356 is similar to the PCD cutting table 150 (Figure 1) and extends from the end of the leached layer 354 to the opposing surface 314. In the unleached layer 356, the cobalt 214 (Figure 2) remains within the interstitial spaces 212 (Figure 2). Although a boundary line 355 is formed between the leached layer 354 and the unleached layer 356 and is depicted as being substantially linear, the boundary line 355 can be non-linear.

The leached PDC cutters 300 are leached to different desired depths 353 and how deep the cutter 300 has been leached has an effect on the performance of the cutter 300. Conventionally, the leached depth 353 of the cutter 300 is measured, or determined, by cutting the cutter 300 vertically in half and then subsequently polishing the cutter 300. The leached depth 353 is visually measured under a microscope or similar magnifying device. This process is rather tedious and cumbersome as it involves cutting the cutter 300, such as by electrical discharge machining ("EDM"), mounting, grinding, and polishing the cutter 300, and performing an analysis under a microscope. Additionally, this process destroys the cutter 300 from subsequently being used. The leached depth 353 that is determined in this manner is assumed to be the same leached depth in other cutters that were leached in the same batch.

**BRIEF DESCRIPTION OF THE DRAWINGS**

The foregoing and other features and aspects of the invention are best understood with reference to the following description of certain exemplary embodiments, when read in conjunction with the accompanying drawings, wherein:

Figure 1 is a side view of a PDC cutter having a polycrystalline diamond cutting table, or compact, in accordance with the prior art;

Figure 2 is a schematic microstructural view of the PCD cutting table of Figure 1 in accordance with the prior art;

Figure 3 is a cross-section view of a PDC cutter having a PCD cutting table that has been at least partially leached in accordance with the prior art;

Figure 4 is a schematic view of a capacitance measuring system in accordance to one exemplary embodiment of the present invention;

Figure 5 is a schematic view of a capacitance measuring system in accordance to another exemplary embodiment of the present invention;

Figure 6 is a schematic view of an eddy current testing system in accordance to one exemplary embodiment of the present invention;

Figure 7A is a graphical chart showing a plurality of shallow leached amplitude curves and a plurality of shallow leached phase angle shift curves for each of a plurality of shallow leached PDC cutters using the eddy current testing system of Figure 6 in accordance with an exemplary embodiment;

Figure 7B is a graphical chart showing a plurality of deep leached amplitude curves and a plurality of deep leached phase angle shift curves for each of a plurality of deep leached PDC cutters using the eddy current testing system of Figure 6 in accordance with an exemplary embodiment;

Figure 7C shows a graphical chart depicting both the amplitude curves and the phase angle shift curves of Figures 7A and 7B for each of the shallow leached PDC cutters and deep leached PDC cutters using the same scale in accordance with an exemplary embodiment;

Figure 8 is a flowchart depicting a non-destructive leaching depth estimation method in accordance with an exemplary embodiment of the present invention;

Figure 9 is a graphical chart depicting a first calibration curve that shows a relationship between capacitance and actual leaching depth for a plurality of leached components in accordance with an exemplary embodiment of the present invention;

Figure 10A is a graphical chart depicting a second calibration curve that shows a relationship between eddy current and actual leaching depth for a plurality of leached components in accordance with an exemplary embodiment of the present invention;

Figure 10B is a partial view of the graphical chart of Figure 10A in accordance with an exemplary embodiment of the present invention;

Figure 11 is a flowchart depicting a microstructural quality determination method in accordance with an exemplary embodiment of the present invention; and

Figure 12 is a data scattering chart that shows the measured capacitance for a plurality of leached cutters from a same leaching batch in accordance with an exemplary embodiment of the present invention.

The drawings illustrate only exemplary embodiments of the invention and are therefore not to be considered limiting of its scope, as the invention may admit to other equally effective embodiments.

**BRIEF DESCRIPTION OF EXEMPLARY EMBODIMENTS**

The present invention is directed to a non-destructive method and apparatus for measuring the leaching depth within an ultra-hard polycrystalline structure and/or characterizing at least a portion of the ultra-hard polycrystalline structure, such as the ones used in forming polycrystalline diamond compact ("PDC") cutters, using at least capacitance and eddy current measurements. Although the description of exemplary embodiments is provided below in conjunction with a PDC cutter, alternate embodiments of the invention may be applicable to other types of polycrystalline structures including, but not limited to, PCBN cutters. Further, according to some exemplary embodiments, one or more portions of the methods described below is implemented using an electronic measuring device. For example, the capacitance is measured using a capacitance measuring device and the eddy current is measured using an eddy current measuring device. The invention is better understood by reading the following description of nonlimiting, exemplary embodiments with reference to the attached drawings, wherein like parts of each of the figures are identified by like reference characters, and which are briefly described as follows.

Figure 4 is a schematic view of a capacitance measuring system 400 in accordance to one exemplary embodiment of the present invention. Referring to Figure 4, the capacitance measuring system 400 includes a capacitance measuring device 410, the leached PDC cutter 300, a first wire 430, and a second wire 440. Although certain components have been enumerated as being included in the capacitance measuring system 400, additional components are included in other exemplary embodiments. Additionally, although the description provided below has been provided with respect to the leached PDC cutter 300, a different component, such as the PCD cutting table 310 alone or other component that includes another type of leached polycrystalline structure, is used in lieu of the leached PDC cutter 300. Additionally, although the description provided below has been provided with respect to the leached PDC cutter 300, a different component, such as a chemically cleaned leached PDC cutter (not shown), is used in lieu of the leached PDC cutter 300. The chemically cleaned leached PDC cutter has had at least a portion of the by-product materials 398 (Figure 3) removed by using one or more processes described in related application entitled, "Method To Improve The Performance Of A Leached Cutter", which has been mentioned above and incorporated by reference herein. The leached PDC cutter 300 has been previously described with respect to Figure 3 and is not repeated again herein for the sake of brevity.

The capacitance measuring device 410 is a device that measures the capacitance of an energy storage device, which is the leached PDC cutter 300 in the instant exemplary embodiment. Capacitance is a measure of the amount of electric potential energy stored, or separated, for a given electric potential. A common form of energy storage device is a parallel-plate capacitor. In the instant exemplary embodiment, the leached PDC cutter 300 is an example of the parallel-plate capacitor. The capacitance of the energy storage device is typically measured in farads, or nanofarads.

One example of the capacitance measuring device 410 is a multi-meter; however, other capacitance measuring devices known to people having ordinary skill in the art are used in one or more alternative exemplary embodiments. The multi-meter 410 includes a positionable dial 412, a plurality of measurement settings 414, a display 416, a positive terminal 418, and a negative terminal 419. According to some exemplary embodiments, the positionable dial 412 is rotatable in a clockwise and/or counterclockwise manner and is set to one of several available measurement settings 414. In the instant exemplary embodiment, the positionable dial 412 is set to a nanofaraday setting 415 so that the multi-meter 410 measures capacitance values. The display 416 is fabricated using polycarbonate, glass, plastic, or other known suitable material and communicates a measurement value, such as a capacitance value, to a user (not shown) of the multi-meter 410. The positive terminal 418 is electrically coupled to one end of the first wire 430, while the negative terminal 419 is electrically coupled to one end of the second wire 440.

The first wire 430 is fabricated using a copper wire or some other suitable conducting material or alloy known to people having ordinary skill in the art. According to some exemplary embodiments, the first wire 430 also includes a non-conducting sheath (not shown) that surrounds the copper wire and extends from about one end of the copper wire to an opposing end of the cooper wire. The two ends of the copper wire are exposed and are not surrounded by the non-conducting sheath. In some exemplary embodiments, an insulating material (not shown) also surrounds the copper wire and is disposed between the copper wire and the non-conducting sheath. The insulating material extends from about one end of the non-conducting sheath to about an opposing end of the non-conducting sheath. As previously mentioned, one end of the first wire 430 is electrically coupled to the positive terminal 418, while the opposing end of the first wire 430 is electrically coupled to the cutting surface 312 of the leached PDC cutter 300. The opposing end of the first wire 430 is electrically coupled to the cutting surface 312 in one of several methods. In one example, the first wire 430 is electrically coupled to the cutting surface 312 using one or more fastening devices (not shown), such as a clamp, or using an equipment (not shown) that supplies a force to retain the first wire 430 in electrical contact with the cutting surface 312. In another example, a clamp (not shown) is coupled to the opposing end of the first wire 430 and a conducting component (not shown), such as aluminum foil, is coupled to, or placed in contact with, the cutting surface 312. The clamp is electrically coupled to the conducting component, thereby electrically coupling the first wire 430 to the cutting surface 312. Additional methods for coupling the first wire 430 to the cutting surface 312 can be used in other exemplary embodiments.

The second wire 440 is fabricated using a copper wire or some other suitable conducting material or alloy known to people having ordinary skill in the art. According to some exemplary embodiments, the second wire 440 also includes a non-conducting sheath (not shown) that surrounds the copper wire and extends from about one end of the copper wire to an opposing end of the cooper wire. The two ends of the copper wire are exposed and are not surrounded by the non-conducting sheath. In some exemplary embodiments, an insulating material (not shown) also surrounds the copper wire and is disposed between the copper wire and the non-conducting sheath. The insulating material extends from about one end of the non-conducting sheath to an opposing end of the non-conducting sheath. As previously mentioned, one end of the second wire 440 is electrically coupled to the negative terminal 419, while the opposing end of the second wire 440 is electrically coupled to a bottom surface 454, which is similar to the bottom surface 154 (Figure 1), of the leached PDC cutter 300. The second wire 440 is electrically coupled to the bottom surface 454 in a similar manner as the first wire 430 is electrically coupled to the cutting surface 312.

Hence, a circuit 405 is completed using the multi-meter 410, the first wire 430, the leached PDC cutter 300, and the second wire 440. The current is able to flow from the positive terminal 418 of the multi-meter 410 to the cutting surface 312 of the leached PDC cutter 300 through the first wire 430. The current then flows through the leached PDC cutter 300 to the bottom surface 454 of the leached PDC cutter 300. When the multi-meter 410 is turned on, a voltage differential exists between the cutting surface 312 and the bottom surface 454. The current then flows from the bottom surface 454 to the negative terminal 419 of the multi-meter 410 through the second wire 440. The capacitance measurement of the leached PDC cutter 300 is determined when the value displayed on the display 416 reaches a peak value or remains constant for a period of time.

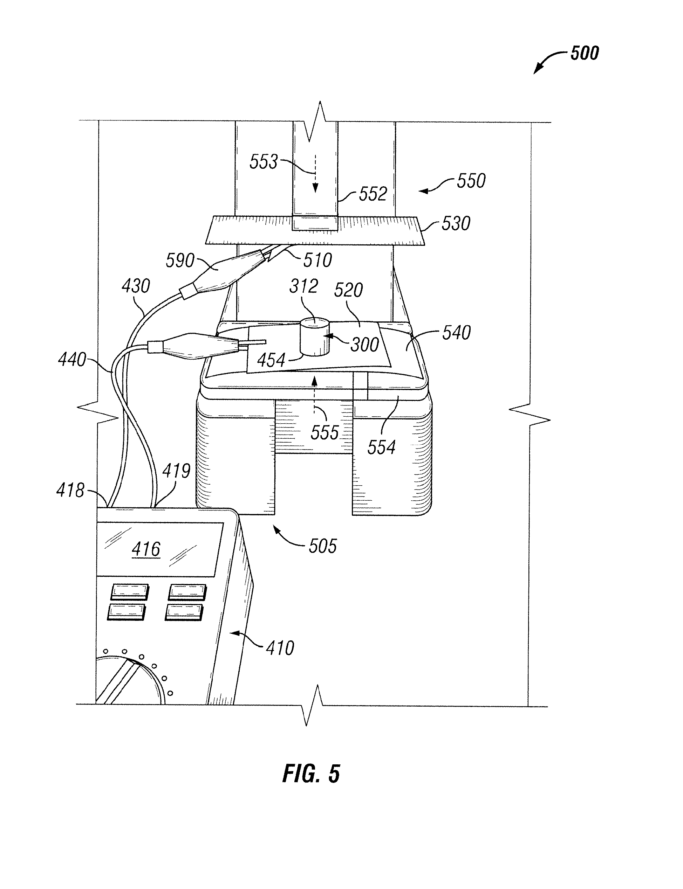

Figure 5 is a schematic view of a capacitance measuring system 500 in accordance to another exemplary embodiment of the present invention. Referring to Figure 5, the capacitance measuring system 500 includes the capacitance measuring device 410, the leached PDC cutter 300, the first wire 430, the second wire 440, a first conducting material 510, a second conducting material 520, a first insulating material 530, a second insulating material 540, and an Arbor Press 550. Although certain components have been enumerated as being included in the capacitance measuring system 500, additional components are included in other exemplary embodiments. Further, although certain components have been enumerated as being included in the capacitance measuring system 500, alternative components having similar functions as the enumerated components are used in alternative exemplary embodiments. Additionally, although the description provided below has been provided with respect to the leached PDC cutter 300, a different component, such as the PCD cutting table 310 (Figure 3) alone or other component that includes another type of leached polycrystalline structure, is used in lieu of the leached PDC cutter 300. Additionally, although the description provided below has been provided with respect to the leached PDC cutter 300, a different component, such as the chemically cleaned leached PDC cutter mentioned above, is used in lieu of the leached PDC cutter 300. The capacitance measuring device 410, the leached PDC cutter 300, the first wire 430, and the second wire 440 have been previously described and are not repeated again herein for the sake of brevity.

The first conducting material 510 and the second conducting material 520 are similar to one another in certain exemplary embodiments, but are different in other exemplary embodiments. According to one exemplary embodiment, the conducting materials 510, 520 are fabricated using aluminum foil; however, other suitable conducting materials can be used. The first conducting material 510 is positioned adjacently above and in contact with the cutting surface 312. The second conducting material 520 is positioned adjacently below and in contact with the bottom surface 454. The first conducting material 510 and the second conducting material 520 provide an area to which the first wire 430 and the second wire 440, respectively, make electrical contact. Additionally, the first conducting material 510 and the second conducting material 520 assist in minimizing contact resistance with the cutting surface 312 and the bottom surface 454, respectively, which is discussed in further detail below. In certain exemplary embodiments, the first conducting material 510 and the second conducting material 520 are the same shape and size; while in other exemplary embodiments, one of the conducting materials 510, 520 is a different shape and/or size than the other conducting material 510, 520.

The first insulating material 530 and the second insulating material 540 are similar to one another in certain exemplary embodiments, but are different in other exemplary embodiments. According to one exemplary embodiment, the insulating materials 530, 540 are fabricated using paper; however, other suitable insulating materials, such as rubber, can be used. The first insulating material 530 is positioned adjacently above and in contact with the first conducting material 510. The second insulating material 540 is positioned adjacently below and in contact with the second conducting material 520. The first insulating material 530 and the second insulating material 540 provide a barrier to direct current flow only through a circuit 505, which is discussed in further detail below. In certain exemplary embodiments, the first insulating material 530 and the second insulating material 540 are the same shape and size; while in other exemplary embodiments, one of the insulating materials 530, 540 is a different shape and/or size than the other insulating material 530, 540. Additionally, in certain exemplary embodiments, the insulating materials 530, 540 is larger in size than its corresponding conducting material 510, 520. However, one or more of the insulating materials 530, 540 is either larger or smaller than its corresponding conducting material 510, 520 in alternative exemplary embodiments.

The Arbor Press 550 includes an upper plate 552 and a base plate 554. The upper plate 552 is positioned above the base plate 554 and is movable towards the base plate 554. In other exemplary embodiments, the base plate 554 is movable towards the upper plate 552. The first insulating material 530, the first conducting material 510, the leached PDC cutter 300, the second conducting material 520, and the second insulating material 540 are positioned between the upper plate 552 and the base plate 554 such that the second insulating material 540 is positioned adjacently above and in contact with the base plate 554. The upper plate 552 is moved towards the base plate 554 until the upper plate 552 applies a downward load 553 onto the cutting surface 312 of the leached PDC cutter 300. When the downward load 553 is applied, the first conducting material 510 is deformed and adapted to the rough and very stiff cutting surface 312, thereby minimizing contact resistance between the first conducting material 510 and the cutting surface 312 and greatly improving the capacitance measurement consistency. At this time, the base plate 554 also applies an upward load 555 onto the bottom surface 454 of the leached PDC cutter 300. When the upward load 555 is applied, the second conducting material 520 is deformed and adapted to the rough and very stiff bottom surface 454, thereby minimizing contact resistance between the second conducting material 520 and the bottom surface 454 and greatly improving the capacitance measurement consistency. In certain exemplary embodiments, the downward load 553 is equal to the upward load 555. The downward load 553 and the upward load 555 is about one hundred pounds; however, these loads 553, 555 range from about two pounds to about a critical load. The critical load is a load at which the leached PDC cutter 300 is damaged when applied thereto.

In one exemplary embodiment, the second insulating material 540 is positioned on the base plate 554, the second conducting material 520 is positioned on the second insulating material 540, the leached PDC cutter 300 is positioned on the second conducting material 520, the first conducting material 510 is positioned on the leached PDC cutter 300, and the first insulating material 530 is positioned on the first conducting material 510. The upper plate 552 is moved towards the first insulating material 530 until the downward load 553 is applied onto the leached PDC cutter 300. In an alternative exemplary embodiment, one or more components, such as the first insulating material 530 and the first conducting material 510, are coupled to the upper plate 552 prior to the upper plate 552 being moved towards the base plate 554. Although an Arbor Press 550 is used in the capacitance measuring system 500, other equipment capable of delivering equal and opposite loads to each of the cutting surface 312 and the bottom surface 454 of the leached PDC cutter 300 can be used in other exemplary embodiments.

One end of the first wire 430 is electrically coupled to the positive terminal 418 of the multi-meter 410, while the opposing end of the first wire 430 is electrically coupled to the first conducting material 510, which thereby becomes electrically coupled to the cutting surface 312 of the leached PDC cutter 300. In one exemplary embodiment, a clamp 590 is coupled to the opposing end of the first wire 430 which couples the first wire 430 to the first conducting material 510. One end of the second wire 440 is electrically coupled to the negative terminal 419 of the multi-meter 410, while the opposing end of the second wire 440 is electrically coupled to the second conducting material 520, which thereby becomes electrically coupled to the bottom surface 454 of the leached PDC cutter 300. In one exemplary embodiment, a clamp (not shown), similar to clamp 590, is coupled to the opposing end of the second wire 440, which couples the second wire 440 to the second conducting material 520. Hence, the circuit 505 is completed using the multi-meter 410, the first wire 430, the first conducting material 510, the leached PDC cutter 300, the second conducting material 520, and the second wire 440. The current is able to flow from the positive terminal 418 of the multi-meter 410 to the cutting surface 312 of the leached PDC cutter 300 through the first wire 430 and the first conducting material 510. The current then flows through the leached PDC cutter 300 to the bottom surface 454 of the leached PDC cutter 300. When the multi-meter 410 is turned on, a voltage differential exists between the cutting surface 312 and the bottom surface 454. The current then flows from the bottom surface 454 to the negative terminal 419 of the multi-meter 410 through the second conducting material 520 and the second wire 440. The first insulating material 530 and the second insulating material 540 prevent the current from flowing into the Arbor Press 550. The capacitance measurement of the leached PDC cutter 300 is determined when the value displayed on the display 416 reaches a peak value or remains constant for a period of time.

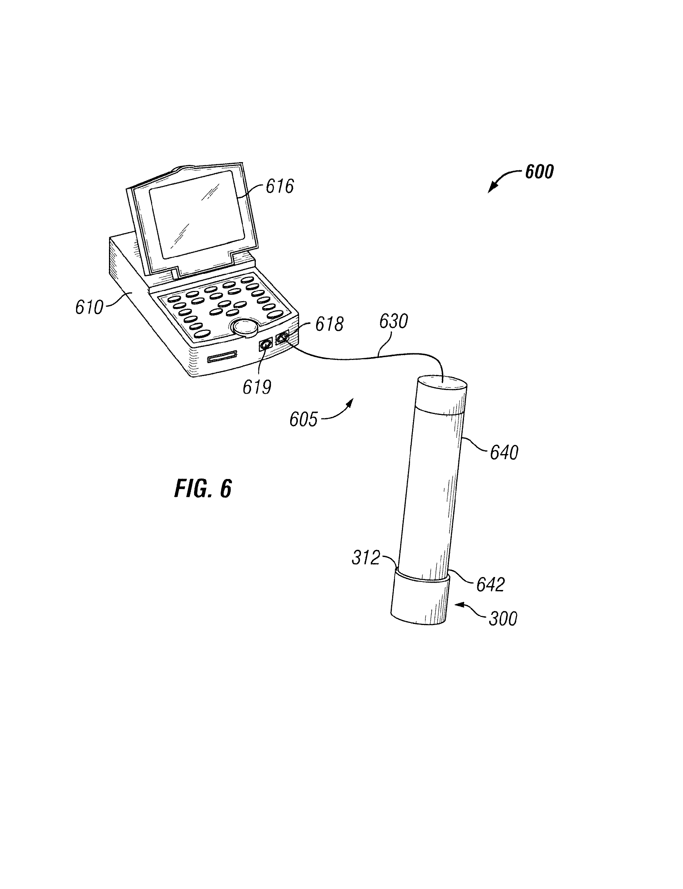

Figure 6 is a schematic view of an eddy current testing system 600 in accordance to one exemplary embodiment of the present invention. Referring to Figure 6, the eddy current testing system 600 includes an eddy current testing device 610, the leached PDC cutter 300, a first wire 630, and a probe 640, which collectively form a circuit 605 (described below) when electrically coupled to one another. Although certain components have been enumerated as being included in the eddy current testing system 600, additional components are included in other exemplary embodiments. Additionally, although the description provided below has been provided with respect to the leached PDC cutter 300, a different component, such as the PCD cutting table 310 alone or other component that includes another type of leached polycrystalline structure, is used in lieu of the leached PDC cutter 300. Additionally, although the description provided below has been provided with respect to the leached PDC cutter 300, a different component, such as a chemically cleaned leached PDC cutter (not shown), is used in lieu of the leached PDC cutter 300. The chemically cleaned leached PDC cutter has had at least a portion of the by-product materials 398 (Figure 3) removed by using one or more processes described in related application entitled, "Method To Improve The Performance Of A Leached Cutter", which has been mentioned above and incorporated by reference herein. The leached PDC cutter 300 has been previously described with respect to Figure 3 and is not repeated again herein for the sake of brevity.

The eddy current testing device 610 is a device that measures eddy currents, which includes at least one of an impedance amplitude and/or a phase angle shift, of the circuit 605 through which alternating current ("AC current") travels. The eddy current testing device 610 includes at least a display 616 and a first terminal 618. The display 616 is fabricated using polycarbonate, glass, plastic, or other known suitable material and communicates one or more measurement values, such as the impedance amplitude and/or the phase angle shift, to a user (not shown) of the eddy current testing device 610. The first terminal 618 is electrically coupled to one end of the first wire 630 and supplies alternating current ("AC current") therethrough. In certain exemplary embodiments, the eddy current testing device 610 includes a power supply terminal 619, which is electrically coupled to a power supply source (not shown). For example, a second wire (not shown) with a plug (not shown) electrically couples the power supply terminal 619 to a wall outlet (not shown), which is a source for alternating current. Alternatively, the eddy current testing device 610 is powered by one or more batteries (not shown) or other known power supply source. The eddy current testing device 610 displays these measurements in graphical form according to some exemplary embodiments, which can be then analyzed to determine the quantitative and/or the qualitative measurements for eddy currents. Alternatively, one or more of the impedance amplitude and/or the phase angle shift measurements are displayed digitally, or quantitatively, on the display 616.

The first wire 630 is fabricated using a copper wire or some other suitable conducting material or alloy known to people having ordinary skill in the art. According to some exemplary embodiments, the first wire 630 also includes a non-conducting sheath (not shown) that surrounds the copper wire and extends from about one end of the copper wire to an opposing end of the cooper wire. The two ends of the copper wire are exposed and are not surrounded by the non-conducting sheath. In some exemplary embodiments, an insulating material (not shown) also surrounds the copper wire and is disposed between the copper wire and the non-conducting sheath. The insulating material extends from about one end of the non-conducting sheath to about an opposing end of the non-conducting sheath. As previously mentioned, one end of the first wire 630 is electrically coupled to the first terminal 618. In certain exemplary embodiments, an adapter (not shown), or other suitable device, is coupled to the one end of the first wire 630 which also is insertable into the first terminal 618. The opposing end of the first wire 630 is electrically coupled to the probe 640.

The probe 640 includes a coil conductor (not shown) therein, which is electrically coupled to the first wire 630. According to some exemplary embodiments, the probe 640 is cylindrically shaped and includes a substantially planar first end 642 which is opposite the end at which the first wire 630 is coupled to the probe 640. However, in other exemplary embodiments, the probe 640 is shaped differently. The first end 642 is positioned in contact with the cutting surface 312 of the leached PDC cutter 300. Thus, the coil conductor transports the alternating current towards the leached PDC cutter 300.

Hence, the circuit 605 is completed using the eddy current testing device 610, the first wire 630, the probe 640, and the leached PDC cutter 300. Once the eddy current testing device 610 is turned on, the alternating current flows from the eddy current testing device 610 to the coil conductor of the probe 640 through the first wire 630. The probe 640 creates a magnetic field. The probe 640 is in close proximity to a second conductor, or the unleached layer 356 (Figure 3) of the leached PDC cutter 300, and is separated by a distance defined by the leached layer 354 (Figure 3) of the leached PDC cutter 300, which also is referred to as the leached depth 353 (Figure 3). This close proximity induces eddy currents in the unleached layer 356 (Figure 3), or second conductor, which is proportional to the frequency of the alternating current field. The distance that the coil conductor, or probe 640, is from the second conductor 356 (Figure 3), which has been mentioned above, is called liftoff. This distance affects the mutual inductance of the circuit 605 and is related to the impedance amplitude and/or a phase angle shift of the circuit 605, which is discussed in further detail below. Thus, the leached depth 353 (Figure 3) is determinable, either quantitatively and/or qualitatively, from the measured impedance amplitude and/or phase angle shift of the circuit 605. Further, the quality of leaching, i.e. the removal of catalyst material from within the leached layer 354 (Figure 3), and/or the microstructure characteristics, e.g. the porosity, is determinable, at least qualitatively, from the measured impedance amplitude and/or phase angle shift of the circuit 605. Specifically, when repeated tests on a leached PDC cutter 300 exhibits less scattered results for the measured impedance amplitude and/or phase angle shift of the circuit 605, the leached PDC cutter 300 has a better quality of leaching and/or a better microstructure characteristic.

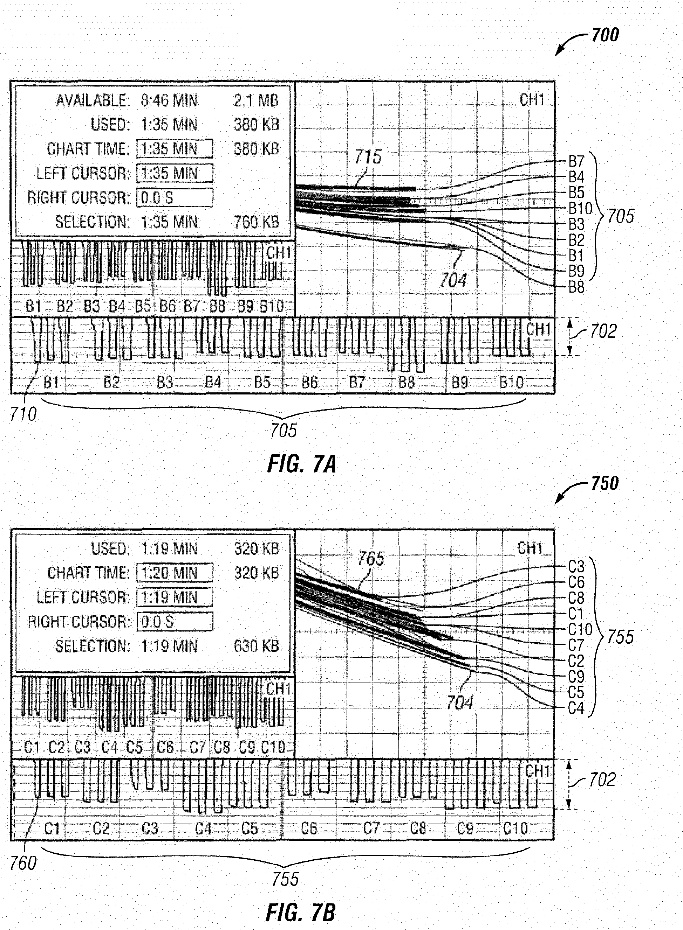

Figure 7A is a graphical chart 700 showing a plurality of shallow leached amplitude curves 710 and a plurality of shallow leached phase angle shift curves 715 for each of a plurality of shallow leached PDC cutters 705 using the eddy current testing system 600 (Figure 6) in accordance with an exemplary embodiment. The shallow leached PDC cutter 705 is similar to the leached PDC cutter 300 (Figure 3), but the leached depth 353 (Figure 3) is a shallow leached depth. A shallow leached depth typically ranges from about forty to seventy microns, but can be greater or lesser than this range in other exemplary embodiments. Referring to Figure 7A, the longitudinal length of each of the shallow leached amplitude curves 710 provides a measurement of the impedance amplitude 702 for the respective shallow leached PDC cutter 705. Similarly, the slope of each of the shallow leached phase angle shift curves 715 provides a measurement of the phase angle shift 704 for the respective shallow leached PDC cutter 705. Typically, the phase angle shift 704 is determined when analyzing the end of the shallow leached phase angle shift curve 715. The graphical chart 700 shows three shallow leached amplitude curves 710 and three shallow leached phase angle shift curves 715 for each of the ten shallow leached PDC cutters 705 that were tested using the eddy current testing system 600 (Figure 6). The impedance amplitude 702 is determined from each of the shallow leached amplitude curve 710, while the phase angle shift 704 is determined from each of the shallow leached phase angle shift curve 715.

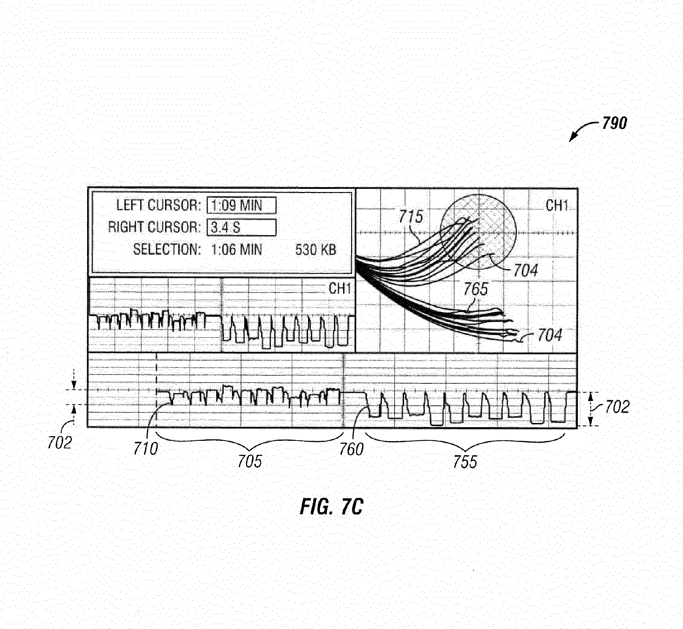

Figure 7B is a graphical chart 750 showing a plurality of deep leached amplitude curves 760 and a plurality of deep leached phase angle shift curves 765 for each of a plurality of deep leached PDC cutters 755 using the eddy current testing system 600 (Figure 6) in accordance with an exemplary embodiment. The deep leached PDC cutter 755 is similar to the leached PDC cutter 300 (Figure 3), but the leached depth 353 (Figure 3) is a deep leached depth. A deep leached depth typically ranges from about 240 to 300 microns, but can be greater or lesser than this range in other exemplary embodiments. Referring to Figure 7B, the longitudinal length of each of the deep leached amplitude curves 760 provides a measurement of the impedance amplitude 702 for the respective deep leached PDC cutter 755. Similarly, the slope of each of the deep leached phase angle shift curves 765 provides a measurement of the phase angle shift 704 for the respective deep leached PDC cutter 755. Typically, the phase angle shift 704 is determined when analyzing the end of the deep leached phase angle shift curve 765. The graphical chart 750 shows three deep leached amplitude curves 760 and three deep leached phase angle shift curves 765 for each of the ten deep leached PDC cutters 755 that were tested using the eddy current testing system 600 (Figure 6). The impedance amplitude 702 is determined from each of the deep leached amplitude curve 760, while the phase angle shift 704 is determined from each of the deep leached phase angle shift curve 765. Although Figures 7A and 7B show the test results for shallow leached PDC cutters 705 and deep leached PDC cutters 755, respectively, these test results are not comparable with one another since the scales used in depicting the amplitude curves 710, 760 and the phase angle shift curves 715, 765 are different for each of the figures. Hence, Figure 7C shows a graphical chart 790 depicting both the amplitude curves 710, 760 and the phase angle shift curves 715, 765 of Figures 7A and 7B for each of the shallow leached PDC cutters 705 and deep leached PDC cutters 755 using the same scale in accordance with an exemplary embodiment. According to Figure 7C, the impedance amplitude 702 for the shallow leached amplitude curves 710 is smaller than the impedance amplitude 702 for the deep leached amplitude curves 760. Conversely, the phase angle shift 704 for the shallow leached phase angle shift curves 715 is larger than the phase angle shift 704 for the deep leached phase angle shift curves 765.

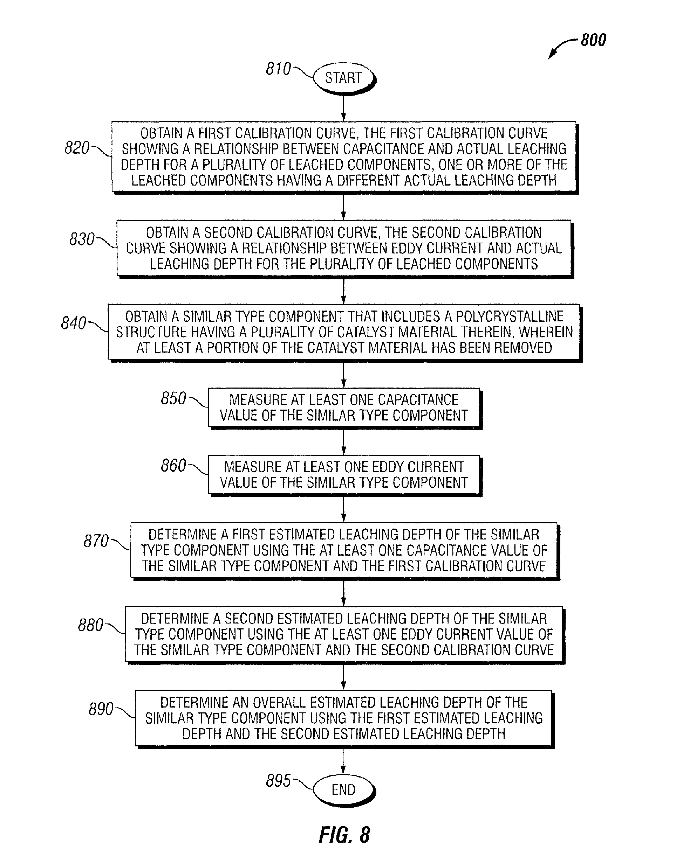

Figure 8 is a flowchart depicting a non-destructive leaching depth estimation method 800 in accordance with an exemplary embodiment of the present invention. Although Figure 8 shows a series of steps depicted in a certain order, the order of one or more steps can be rearranged, combined into fewer steps, and/or separated into more steps than that shown in other exemplary embodiments. Referring to Figure 8, the non-destructive leaching depth estimation method 800 begins at step 810. Upon starting at step 810, the non-destructive leaching depth estimation method 800 proceeds to step 820. At step 820, a first calibration curve is obtained. The first calibration curve can be generated from tests or acquired from elsewhere.

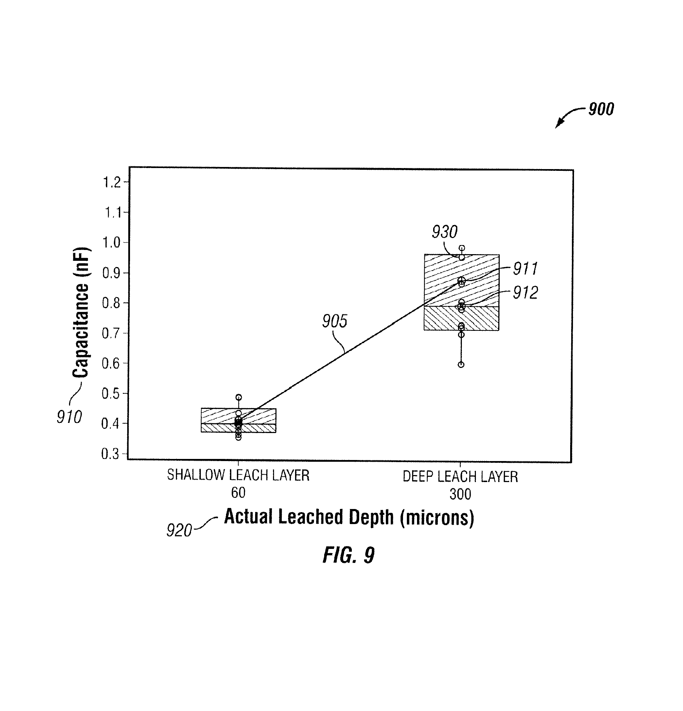

Figure 9 is a graphical chart 900 depicting the first calibration curve 905 that shows a relationship between capacitance 910 and actual leaching depth 920 for a plurality of leached components 300 (Figure 3) in accordance with an exemplary embodiment of the present invention. Referring to Figure 9, one or more of the leached components 300 (Figure 3) have a different actual leaching depth 920 than at least one other leached component 300 (Figure 3). The leached component 300 (Figure 3) is the leached PDC cutter 300 (Figure 3) according to some exemplary embodiments; however, the leached component 300 can be only the PCD cutting table 310 (Figure 3) or some other component that has a polycrystalline structure that has had at least some of the catalyst material removed from therein. Alternatively, in certain exemplary embodiments, the leached component 300 (Figure 3) can be the chemically cleaned leached PDC cutter mentioned above.

The first calibration curve 905 is generated by obtaining two or more leached components 300 (Figure 3). The first calibration curve 905 becomes more precise as more leached components 300 (Figure 3) are used in generating the first calibration curve 905. The capacitance data points 930 are obtained by measuring the capacitance 910 of each leached component 300 (Figure 3). In certain exemplary embodiments, a plurality of capacitance data points 930 are obtained for each leached component 300 (Figure 3). For example, the capacitance 910 is measured five times for each leached component 300 (Figure 3). Obtaining a plurality of capacitance data points 930 for each leached component 300 (Figure 3) improves the statistical significance of the capacitance data points 930 being collected. According to some exemplary embodiments, the leached component 300 (Figure 3) is depolarized after each measurement for capacitance 910, before each measurement for capacitance 910, or before and after each measurement for capacitance 910. The leached component 300 (Figure 3) is depolarized in one or a combination of different manners, such as grounding the leached component 300 (Figure 3), wrapping the leached component 300 (Figure 3) in aluminum foil or similar type material, heat treating the leached component 300 (Figure 3), dropping the leached component 300 (Figure 3) in a salt solution, or waiting to discharge the leached component 300 (Figure 3). The leached component 300 (Figure 3) is discharged by waiting about twenty-four hours, but the waiting time is greater or less in other exemplary embodiments. Depolarizing an object is known to people having ordinary skill in the art.

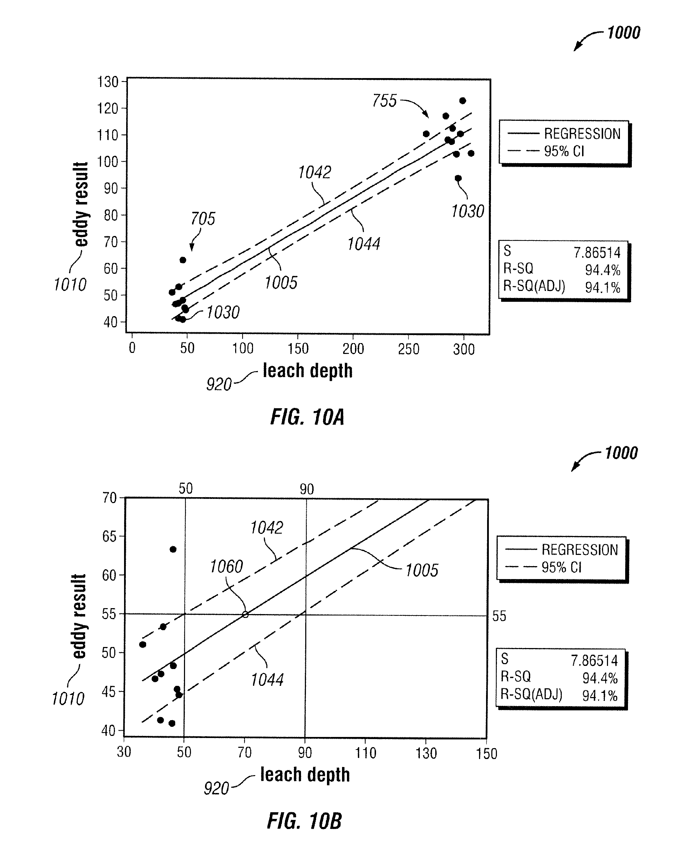

Once the capacitance 910 is measured for each leached component 300 (Figure 3), the eddy current 1010 (Figure 10A), which is discussed in further detail below, is measured before determining the actual leaching depth 920 for each leached component 300 (Figure 3). However, in other exemplary embodiments, the actual leaching depth 920 is determined before measuring the eddy current 1010 (Figure 10A) and a different set of leached components 300 (Figure 3) are used when measuring the eddy current 1010 (Figure 10A) when generating a second calibration curve 1005 (Figure 10A), which is discussed in further detail below. In some examples, the actual leaching depth 920 for a leached component 300 (Figure 3) is determined by cutting the leached component 300 (Figure 3), polishing the cut edge of the leached component 300 (Figure 3), and visually measuring the actual leaching depth 920 under a magnifying device (not shown), such as a microscope. Although one method for determining the actual leaching depth 920 is described, other methods known to people having ordinary skill in the art can be used to determine the actual leaching depth 920 without departing from the scope and spirit of the exemplary embodiment. Each capacitance data point 930 is plotted on the graphical chart 900, where the actual leaching depth 920 is plotted versus the capacitance 910 that is measured. Once the capacitance data points 930 are plotted on the graphical chart 900, the first calibration curve 905 is determined pursuant to methods known to people having ordinary skill in the art. For example, the first calibration curve 905 is generated by using the average capacitance 911 of each leached component 300, the median capacitance 912 of each leached component, or by calculating the best fit curve. The best fit curve can be formed with a ninety-five percent confidence level, but this confidence level can range from about sixty percent to almost about one hundred percent, for example, 99.99 percent. The first calibration curve 905 correlates the measured capacitance 910, which can be measured in nanofarads, with the actual leaching depth 920, which can be measured in microns. Although a few methods for generating the first calibration curve 905 have been described, other methods, either destructive or non-destructive, can be used to generate the first calibration curve 905.

According to Figure 9, the actual leaching depth 920 is directly related to the capacitance 910. Thus, as the actual leaching depth 920 increases, the capacitance 910 that is measured also increases. Conversely, as the actual leaching depth 920 decreases, the capacitance 910 that is measured also decreases. Additionally, the data scattering, or range of measured capacitance 910, is greater as the actual leaching depth 920 increases. Although Figure 9 shows a direct relationship between the actual leaching depth 920 and the capacitance 910; in actuality, the relationship between the capacitance 910 and the actual leaching depth 920 is an inverse relationship. The formula to calculate the capacitance 910 is:

$$C = \varepsilon_r \left( A / (4 \pi d) \right)$$

where
C is the capacitance;
A is the area of overlap of the two plates;
$\varepsilon_T$ is the relative static permittivity (sometimes called the dielectric constant); and
d is the separation between the plates.
Thus, as "d", or the actual leaching depth 920, increases, the capacitance 910 decreases, and visa versa. The opposite phenomena is occurring in Figure 9 because the by-product materials 398 (Figure 3) present with the leached layer 354 (Figure 3) becomes polarized during the measurements, and thus the relative static permittivity is not constant.

Therefore, in certain exemplary embodiments, the leached layer 354 (Figure 3) is treated, such as by chemical treatment, to have at least a portion of the by-product materials 398 (Figure 3) removed. This treatment is dependent upon the methods and/or chemicals used to leach the PCD cutting table 310 (Figure 3). This treated leached PDC cutter is used within the capacitance measuring system 400, 500 (Figures 4 and 5, respectively) or within some other capacitance measuring system in lieu of the leached PDC cutter 300 (Figure 3). The calibration curve that is determined using the treated leached PDC cutters would show the relationship between the actual leaching depth 920 and the capacitance 910 being an inverse relationship. In the methods using the treated leached PDC cutter, which has had at least a portion of the by-product materials 398 (Figure 3) removed, the depolarizing step is optional.

Referring back to Figure 8, the non-destructive leaching depth estimation method 800 proceeds to step 830. At step 830, a second calibration curve is obtained. The second calibration curve can be generated from tests or acquired from elsewhere.

Figure 10A is a graphical chart 1000 depicting the second calibration curve 1005 that shows a relationship between eddy current 1010 and actual leaching depth 920 for a plurality of leached components 300 (Figure 3) in accordance with an exemplary embodiment of the present invention. The eddy current 1010 is the impedance amplitude 702 (Figures 7A-7C) in the exemplary embodiment shown in Figure 10A, however, the phase angle shift 704 (Figures 7A-7C) can be used for the eddy current 1010 in lieu of the impedance amplitude 702 (Figures 7A-7C) in other exemplary embodiments. Referring to Figure 10A, one or more of the leached components 300 (Figure 3) have a different actual leaching depth 920 than at least one other leached component 300 (Figure 3). In the exemplary embodiment shown, the leached components 300 (Figure 3) include shallow leached PDC cutters 705 and deep leached PDC cutters 755 of Figures 7A-7C. The leached component 300 (Figure 3) is the leached PDC cutter 300 (Figure 3) according to some exemplary embodiments; however, the leached component 300 can be only the PCD cutting table 310 (Figure 3) or some other component that has a polycrystalline structure that has had at least some of the catalyst material removed from therein. Alternatively, in certain exemplary embodiments, the leached component 300 (Figure 3) can be the chemically cleaned leached PDC cutter mentioned above. Therefore, in these alternative exemplary embodiments, the leached layer 354 (Figure 3) is treated, such as by chemical treatment, to have at least a portion of the by-product materials 398 (Figure 3) removed. This treatment is dependent upon the methods and/or chemicals used to leach the PCD cutting table 310 (Figure 3). This treated leached PDC cutter is used within the eddy current testing system 600 (Figure 6) or within some other eddy current testing system in lieu of the leached PDC cutter 300 (Figure 3) in accordance with some exemplary embodiments. In the methods using the treated leached PDC cutter, which has had at least a portion of the by-product materials 398 (Figure 3) removed, the demagnetizing step is optional.

The second calibration curve 1005 is generated by obtaining two or more leached components 300 (Figure 3). The second calibration curve 1005 becomes more precise as more leached components 300 (Figure 3) are used in generating the second calibration curve 1005. The eddy current data points 1030 are obtained by measuring the eddy current 1010, either the impedance amplitude 702 (Figures 7A-7C) and/or the phase angle shift 704 (Figures 7A-7C), of each leached component 300 (Figure 3). In certain exemplary embodiments, a plurality of eddy current data points 1030 are obtained for each leached component 300 (Figure 3), which has been illustrated in Figures 7A-7C. For example, the eddy current 1010 is measured three times for each leached component 300 (Figure 3). Obtaining a plurality of eddy current data points 1030 for each leached component 300 (Figure 3) improves the statistical significance of the eddy current data points 1030 being collected. According to some exemplary embodiments, the leached component 300 (Figure 3) is demagnetized after each measurement for eddy current 1010, before each measurement for eddy current 1010, or before and after each measurement for eddy current 1010. The leached component 300 (Figure 3) is demagnetized in one or a combination of different manners, such as heating the cutters above the curie temperature of the catalyst, or cobalt, which is about 1115 °C, running the cutters through an alternating current field, grounding the leached component 300 (Figure 3), wrapping the leached component 300 (Figure 3) in aluminum foil or similar type material, dropping the leached component 300 (Figure 3) in a salt solution, or waiting a period of time to discharge the leached component 300 (Figure 3). The leached component 300 (Figure 3) is demagnetized by waiting about twenty-four hours, but the waiting time is greater or less in other exemplary embodiments. Methods for demagnetizing an object is known to people having ordinary skill in the art.

Once the eddy current 1010 is measured for each leached component 300 (Figure 3), the actual leaching depth 920 for each leached component 300 (Figure 3) is determined. In some examples, the actual leaching depth 920 for a leached component 300 (Figure 3) is determined by cutting the leached component 300 (Figure 3), polishing the cut edge of the leached component 300 (Figure 3), and visually measuring the actual leaching depth 720 under a magnifying device (not shown), such as a microscope. Although one method for determining the actual leaching depth 920 is described, other methods known to people having ordinary skill in the art can be used to determine the actual leaching depth 920 without departing from the scope and spirit of the exemplary embodiment. Each eddy current data point 1030 is plotted on the graphical chart 1000, where the actual leaching depth 920 is plotted versus the eddy current 1010 that is measured. In Figure 10A, the eddy current 1010 that is measured is the impedance amplitude 702 (Figures 7A-7C). Once the eddy current data points 1030 are plotted on the graphical chart 1000, the second calibration curve 1005 is determined pursuant to methods known to people having ordinary skill in the art. For example, the second calibration curve 1005 is generated by using the average eddy current of each leached component 300 (Figure 3), the median eddy current of each leached component 300 (Figure 3), or by calculating the best fit curve. The best fit curve can be formed with a ninety-five percent confidence level, but this confidence level can range from about sixty percent to almost about one hundred percent, for example, 99.99 percent.

In Figure 10A, an upper 95% confidence line 1042 and a lower 95% confidence line 1044 are illustrated above and below the second calibration curve 1005. These upper 95% confidence line 1042 and lower 95% confidence line 1044 provide a range for estimating the actual leaching depth 920 from the measured eddy current 1010 at a 95% confidence level, which is discussed in further detail with respect to Figure 10B below. The second calibration curve 1005 correlates the measured eddy current 1010 with the actual leaching depth 920, which can be measured in microns. Although a few methods for generating the second calibration curve 1005 have been described, other methods, either destructive or non-destructive, can be used to generate the second calibration curve 1005.

According to Figure 10A, the actual leaching depth 920 is directly related to the impedance amplitude 702 (Figure 7A), which is labeled as eddy current 1010 in Figure 10A. Thus, as the actual leaching depth 920 increases, the eddy current 1010, or impedance amplitude 702 (Figure 7A), that is measured also increases. Conversely, as the actual leaching depth 920 decreases, the eddy current 1010, or impedance amplitude 702 (Figure 7A), that is measured also decreases. Although a direct relationship exists between the actual leaching depth 920 and the impedance amplitude 702 (Figure 7A), conversely, an indirect relationship exists between the actual leaching depth 920 and the phase angle shift 704 (Figure 7A).

Referring back to Figure 8, the non-destructive leaching depth estimation method 800 proceeds to step 840. At step 840, a similar type component, similar to leached cutter 300 (Figure 3), is obtained. However, if the first and second calibration curves 905, 1005 (Figures 9 and 10, respectively) were determined using treated leached PDC cutters, the similar type component is a different treated leached PDC cutter where the actual leaching depth is desired to be ascertained. This similar type component includes a polycrystalline structure that has a plurality of catalyst material therein. At least a portion of this catalyst material has been removed. This removed portion has an unknown depth, which is the leaching depth.

The non-destructive leaching depth estimation method 800 proceeds to step 850. At step 850, the capacitance of the similar type component is measured. According to some exemplary embodiment, this capacitance is measured using the capacitance measuring system 400 (Figure 4) or the capacitance measuring system 500 (Figure 5).

The non-destructive leaching depth estimation method 800 proceeds to step 860. At step 860, the eddy current of the similar type component is measured. According to some exemplary embodiments, this eddy current is measured using the eddy current testing system 600 (Figure 6), wherein the impedance amplitude and/or the phase angle shift is obtained.

The non-destructive leaching depth estimation method 800 proceeds to step 870. At step 870, a first estimated leaching depth of the similar type component is determined using at least one capacitance value of the similar type component and the first calibration curve 905 (Figure 9). The first estimated leaching depth is an estimation of the actual leaching depth and ranges from about one micron to about fifty microns from the actual leaching depth according to some exemplary embodiments.

The non-destructive leaching depth estimation method 800 proceeds to step 880. At step 880, a second estimated leaching depth of the similar type component is determined using at least one eddy current value of the similar type component and the second calibration curve 1005 (Figure 10A). The second estimated leaching depth is an estimation of the actual leaching depth and ranges from about one micron to about fifty microns from the actual leaching depth according to some exemplary embodiments.

Figure 10B is a partial view of the graphical chart 1000 of Figure 10A in accordance with an exemplary embodiment of the present invention. Referring to Figure 10B, the eddy current 1010 measured with the similar type component is fifty-five according to one exemplary embodiment. The actual leaching depth 920 is obtained by finding a point 1060 along the second calibration curve 1005 where the eddy current 1010 is fifty-five. Thus, when the eddy current 1010 is fifty-five, it is determined that the actual leaching depth 920 for the similar type component is seventy microns. Using the upper and lower 95% confidence lines 1042 and 1044, it is determined that the actual leaching depth 920 ranges from about fifty microns to about ninety microns at a 95% confidence level.

Referring back to Figure 8, the non-destructive leaching depth estimation method 800 proceeds to step 890. At step 890, an overall estimated leaching depth of the similar type component is determined using the first estimated leaching depth and the second estimated leaching depth. According to some exemplary embodiments, the overall estimated leaching depth is determined by averaging the first estimated leaching depth and the second estimated leaching depth. According to another exemplary embodiment, the overall estimated leaching depth is determined by multiplying the first estimated leaching depth with a first multiplier factor and the second estimated leaching depth with a second multiplier factor. According to some exemplary embodiments, these first and second multiplier factors are determined by calculating a best fit curve using the first estimated leaching depth, the second estimated leaching depth, and the actual leaching depth as known to people having ordinary skill in the art having the benefit of the present disclosure. The non-destructive leaching depth estimation method 800 then proceeds to step 895, where the non-destructive leaching depth estimation method 800 ends. As previously mentioned, although the eddy current measurement used in the exemplary embodiment is the impedance amplitude, the phase angle shift can be used as the eddy current measurement in other exemplary embodiments.

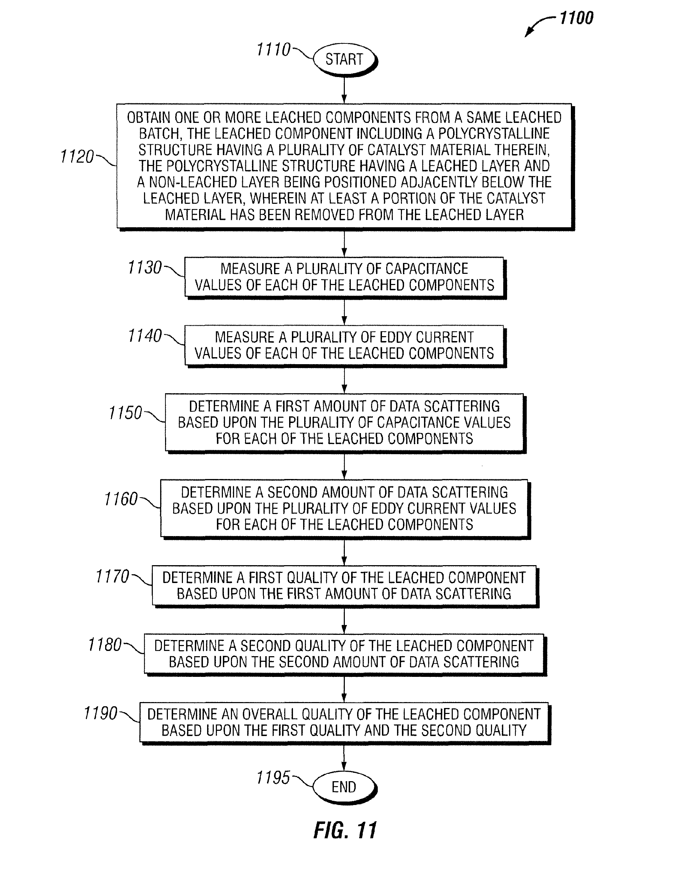

Figure 11 is a flowchart depicting a microstructural quality determination method 1100 in accordance with an exemplary embodiment of the present invention. Although Figure 11 shows a series of steps depicted in a certain order, the order of one or more steps can be rearranged, combined into fewer steps, and/or separated into more steps than that shown in other exemplary embodiments. Referring to Figure 11, the microstructural quality determination method 1100 begins at step 1110. Upon starting at step 1110, the microstructural quality determination method 1100 proceeds to step 1120. At step 1120, one or more leached components that include a polycrystalline structure is obtained from a same leached batch. The same leached batch is a group of components that were leached in the same leaching process at the same time. The polycrystalline structure includes a leached layer and a non-leached layer being positioned adjacently below the leached layer. The non-leached layer includes a plurality of catalyst material therein, while the leached layer has had at least a portion of the catalyst material removed.

The microstructural quality determination method 1100 proceeds to step 1130. At step 1130, a plurality of capacitance values are measured for each of the leached components. The capacitance values are determined using the capacitance measuring system 400 (Figure 4) or the capacitance measuring system 500 (Figure 5).

The microstructural quality determination method 1100 proceeds to step 1140. At step 1140, a plurality of eddy current values are measured for each of the leached components. The eddy current values are determined using the eddy current testing system 600 (Figure 6). The eddy current value measured is the impedance amplitude in some exemplary embodiments; however, in other exemplary embodiments, the phase angle shift is the measured eddy current value.

The microstructural quality determination method 1100 proceeds to step 1150. At step 1150, a first amount of data scattering is determined based upon the plurality of capacitance values for each of the leached components. The first amount of data scattering for a leached component is determined by a differential between the highest measured capacitance and the lowest measured capacitance for that leached component and by statistical results of where each measured capacitance lies.

The microstructural quality determination method 1100 proceeds to step 1160. At step 1160, a second amount of data scattering is determined based upon the plurality of eddy current values for each of the leached components. The second amount of data scattering for a leached component is determined by a differential between the highest measured eddy current value and the lowest measured eddy current value for that leached component and by statistical results of where each measured eddy current value lies. As previously mentioned, the eddy current value is at least one of the impedance amplitude and the phase angle shift.

The microstructural quality determination method 1100 proceeds to step 1170. At step 1170, a first quality of the leached component is determined based upon the first amount of data scattering. The first quality of the leached component relates to the microstructural quality and/or the leaching quality. The microstructural quality relates to the porosity of the microstructure. The microstructural quality is a good quality when there is low porosity. Conversely, the microstructural quality is a poor quality when there is high porosity. The leaching quality is a good quality when there is less catalyst materials present within the leached layer of the polycrystalline structure. Conversely, the leaching quality is a poor quality when there is more catalyst materials present within the leached layer of the polycrystalline structure. In some exemplary embodiments, the first quality of the leached component is considered to be good when the first amount of data scattering is determined to be small. Conversely, the first quality of the leached component is considered to be poor when the first amount of data scattering is determined to be large. The relative terms of small and large are determined when comparing the first amount of data scattering of a first leached component to the first amount of data scattering of a second leached component that was leached in the same batch as the first leached component.

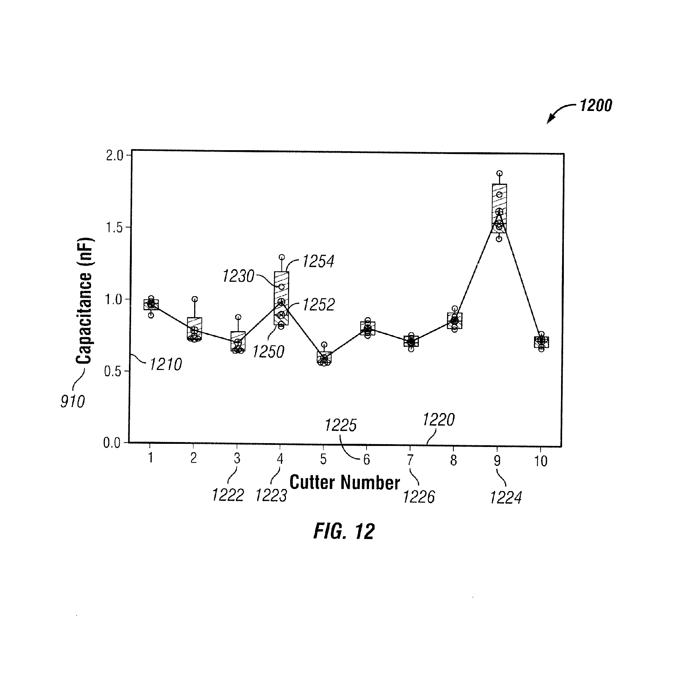

Figure 12 is a data scattering chart 1200 that shows the measured capacitance 910 for a plurality of leached cutters 1222 from a same leaching batch in accordance with an exemplary embodiment of the present invention. Referring to Figure 12, the data scattering chart 1200 includes a cutter number axis 1220 and a capacitance axis 1210. The cutter number axis 1220 includes the number of the cutters 1222 tested. The capacitance axis 1210 includes values for the measured capacitance 910. A capacitance data point 1230 is obtained by measuring the capacitance of the cutter 1222, or leached component 1222, using the capacitance measuring system 400 (Figure 4), the capacitance measuring system 500 (Figure 5), or a similar type system. Each capacitance data point 1230 is plotted on the data scattering chart 1200. Each leached component 1222 has its capacitance measured a plurality of times. In some exemplary embodiments, five capacitance data points 1230 are obtained for each leached component 1222, however, the number of measurements is greater or fewer in other exemplary embodiments. In some exemplary embodiments, a twenty-five percentile marking 1250, a fifty percentile marking 1252 (or average), and a seventy-five percentile marking 1254 is shown in the chart 1200 for each leached component 1222,. The area between the twenty-five percentile marking 1250 and the seventy-five percentile marking 1254 is shaded. The first amount of data scattering is ascertained using this data scattering chart 1200 and can be one or more of a differential between the highest and lowest capacitance measurements 910 for each leached component 1222, a range between the twenty-five percentile marking 1250 and the seventy-five percentile marking 1254, or some similar observation made from the data scattering chart 1200.

According to Figure 12, cutter number 4 1223 and cutter number 9 1224 have a larger first amount of data scattering than for example cutter number 6 1225 or cutter number 7 1226. Hence, cutter number 4 1223 and cutter number 9 1224 have a poor leaching quality and/or a poor microstructural quality within the polycrystalline structure when compared to cutter number 6 1225 or cutter number 7 1226. The increase in amount of catalyst material within the polycrystalline structure causes this data scattering.

Referring back to Figure 11, the microstructural quality determination method 1100 proceeds to step 1180. At step 1180, a second quality of the leached component is determined based upon the second amount of data scattering. The second quality of the leached component relates to the microstructural quality and/or the leaching quality. The microstructural quality relates to the porosity of the microstructure. The microstructural quality is a good quality when there is low porosity. Conversely, the microstructural quality is a poor quality when there is high porosity. The leaching quality is a good quality when there is less catalyst materials present within the leached layer of the polycrystalline structure. Conversely, the leaching quality is a poor quality when there is more catalyst materials present within the leached layer of the polycrystalline structure. In some exemplary embodiments, the second quality of the leached component is considered to be good when the second amount of data scattering is determined to be small. Conversely, the second quality of the leached component is considered to be poor when the second amount of data scattering is determined to be large. The relative terms of small and large are determined when comparing the second amount of data scattering of a first leached component to the second amount of data scattering of a second leached component that was leached in the same batch as the first leached component.

Referring back to Figure 10A, the second amount of data scattering is seen for both shallow leached PDC cutters 705 and deep leached PDC cutters 755. Shallow leached PDC cutters 705 have less second amount of data scattering than deep leached PDC cutters 755. As shown, there are three out of ten shallow leached PDC cutters 705 that have eddy current values that fall outside of the range defined by the upper 95% confidence line 1042 and the lower 95% confidence line 1044, while there are six out of ten deep leached PDC cutters 755 that have eddy current values that fall outside of the range defined by the upper 95% confidence line 1042 and the lower 95% confidence line 1044. Hence, the shallow leached PDC cutters 705 have a better microstructural quality and/or leaching quality than the deep leached PDC cutters 755.

Referring back to Figure 11, the microstructural quality determination method 1100 proceeds to step 1190. At step 1190, an overall quality of the leached component is determined based upon the first quality and the second quality. According to some exemplary embodiments, the overall quality is good when the results of the first quality and the second quality are both good. The overall quality is average when the results of one of the first quality and the second quality is good and the results of the other is poor. The overall quality is poor when the results of the first quality and the second quality are both poor. The microstructural quality determination method 1100 then proceeds to step 1195, where the microstructural quality determination method 1100 ends. As previously mentioned, although the eddy current measurement used in the exemplary embodiment is the impedance amplitude, the phase angle shift can be used as the eddy current measurement in other exemplary embodiments.

According to some exemplary embodiments, the combined use of the capacitance and eddy current measuring techniques help reduce the statistical noise, thereby providing a better resolution. In certain examples, the resolution is about plus/minus five to ten microns, but the range is different in other exemplary embodiments. Combining the two techniques also help in separating the effect of the residual metal binder from the effect of the leaching depth. The two techniques are not used at the same time; but instead, are used sequentially. In certain exemplary embodiments, the capacitance measuring technique is used prior to the eddy current measuring technique; however, in other exemplary embodiments, the eddy current measuring technique is used prior to the capacitance measuring technique.

There are several benefits for non-destructively determining the leaching depth in an ultra-hard polycrystalline structure and/or characterizing at least a portion of the ultra-hard polycrystalline structure. For example, eddy current measurements and/or capacitance measurements can be made on all PDC cutters that are to be mounted and used in a tool, such as a drill bit, thereby being able to estimate the leaching depth in the ultra-hard polycrystalline structure included in the PDC cutter and/or characterizing at least a portion of the ultra-hard polycrystalline structure, such as the quality of the leaching and/or the quality of the microstructure. Hence, only certain PDC cutters are chosen to be mounted to the drill bit or other downhole tool. In another example, when a quantity of PDC cutters being leached within the same leaching batch are provided, such as one thousand PDC cutters, the eddy current and/or capacitance of the PDC cutters are measured pursuant to the descriptions provided above. The PDC cutters that meet a desired quality and/or leaching depth are kept while the remaining PDC cutters that do not meet the desired leaching depth and/or quality are returned. Thus, in one exemplary embodiment, although one thousand PDC cutters being leached from the same batch are provided, two hundred PDC cutters, or twenty percent, may be retained while the remaining are returned. Thus, only the higher quality and/or the proper leaching depth PDC cutters are paid for and retained, which results in the PDC cutters performing better during their application.

Although each exemplary embodiment has been described in detail, it is to be construed that any features and modifications that are applicable to one embodiment are also applicable to the other embodiments. Furthermore, although the invention has been described with reference to specific embodiments, these descriptions are not meant to be construed in a limiting sense. Various modifications of the disclosed embodiments, as well as alternative embodiments of the invention will become apparent to persons of ordinary skill in the art upon reference to the description of the exemplary embodiments. It should be appreciated by those of ordinary skill in the art that the conception and the specific embodiments disclosed may be readily utilized as a basis for modifying or designing other structures or methods for carrying out the same purposes of the invention. It should also be realized by those of ordinary skill in the art that such equivalent constructions do not depart from the spirit and scope of the invention as set forth in the appended claims. It is therefore, contemplated that the claims will cover any such modifications or embodiments that fall within the scope of the invention.

The following are particularly preferred aspects according to the present disclosure.

Clause 1. A method of characterizing a quality of a polycrystalline structure, comprising: obtaining a leached component comprising a polycrystalline structure, the polycrystalline structure comprising a leached layer and an unleached layer positioned adjacent to the leached layer, the leached layer having at least a portion of a catalyst material removed from therein; measuring at least one measured capacitance value of the leached component; measuring at least one measured eddy current value of the leached component; characterizing a first quality of the polycrystalline structure, the first quality being based upon the at least one measured capacitance value; characterizing a second quality of the polycrystalline structure, the second quality being based upon the at least one measured eddy current value; and determining an overall quality of the polycrystalline structure, the overall quality being based upon the first quality and the second quality.

Clause 2. The method of clause 1, further comprising: obtaining a first calibration curve showing a relationship between a plurality of capacitance values and a plurality of actual leaching depth within the polycrystalline structure; and obtaining a second calibration curve showing a relationship between a plurality of eddy current values and the plurality of actual leaching depth within the polycrystalline structure; wherein characterizing the first quality of the polycrystalline structure comprises using the one or more measured capacitance values of the leached component and the first calibration curve to estimate a first actual leaching depth within the leached component, and wherein characterizing the second quality of the polycrystalline structure comprises using the one or more measured eddy current values of the leached component and the second calibration curve to estimate a second actual leaching depth within the leached component.

Clause 3. The method of any of clauses 1 or 2, wherein the overall quality comprises averaging the first actual leaching depth and the second actual leaching depth.

Clause 4. The method of any of clauses 1 to 3, wherein the one or more measured eddy current value comprises at least one of an impedance amplitude and a phase angle shift.

Clause 5. The method of any of clauses 1 to 4, further comprising: obtaining a first calibration curve showing a relationship between a plurality of capacitance values and a plurality of actual leaching depth within the polycrystalline structure; and obtaining a second calibration curve showing a relationship between a plurality of eddy current values and the plurality of actual leaching depth within the polycrystalline structure, wherein characterizing the first quality of the polycrystalline structure comprises: determining a first average of the at least one measured capacitance value of the leached component;

and using the first average and the first calibration curve to estimate a first actual leaching depth within the leached component, and wherein characterizing the second quality of the polycrystalline structure comprises: determining a second average of the at least one measured eddy current value of the leached component; and using the second average and the second calibration curve to estimate a second actual leaching depth within the leached component.

Clause 6. The method of any of clauses 1 to 5, wherein the overall quality comprises averaging the first actual leaching depth and the second actual leaching depth.

Clause 7. The method of any of clauses 1 to 6, wherein the one or more measured eddy current value comprises at least one of an impedance amplitude and a phase angle shift.

Clause 8. The method of any of clauses 1 to 7, wherein the one or more measured eddy current value comprises at least one of an impedance amplitude and a phase angle shift.

Clause 9. The method of any of clauses 1 to 8, further comprising depolarizing the leached component.

Clause 10. The method of clause 9, wherein depolarizing the leached component is performed in at least one of before measuring each measured capacitance value and after measuring each measured capacitance value and in at least one of before measuring each measured eddy current value and after measuring each measured eddy current value.

Clause 11. The method of clause 9, wherein depolarizing the leached component comprises at least one of grounding the leached component, wrapping the leached component in a depolarizing material, heat treating the leached component, placing the leached component in a salt solution, and waiting a period of time.

Clause 12. The method of any of clauses 1 to 11, further comprising cleaning the polycrystalline structure from one or more by-product materials, wherein the by-product materials were deposited within the polycrystalline structure during a leaching process that removes at least a portion of the catalyst material from therein and forms the leached component.

Clause 13. The method of any of clauses 1 to 12, further comprising: measuring a plurality of measured capacitance values for each of a plurality of the leached components, each leached component being formed during a same leaching process; and measuring a plurality of measured eddy current values for each of the plurality of the leached components; wherein characterizing the first quality of the polycrystalline structure comprises using the plurality of measured capacitance values for each leached component to determine a first microstructure quality of the polycrystalline structure for each leached component, and wherein characterizing the second quality of the polycrystalline structure comprises using the plurality of measured eddy current values for each leached component to determine a second microstructure quality of the polycrystalline structure for each leached component.

Clause 14. The method of clause 13, further comprising: determining a first data scattering range for each of the plurality of the leached components from the plurality of measured capacitance values; and determining a second data scattering range for each of the plurality of the leached components from the plurality of measured eddy current values, wherein characterizing the first quality of the polycrystalline structure comprises using the first data scattering range to determine the first microstructure quality of the polycrystalline structure for each leached component, the first microstructure quality of the polycrystalline structure being less porous when the first data scattering range is less in comparison to the first data scattering ranges of other leached components, and wherein characterizing the second quality of the polycrystalline structure comprises using the second data scattering range to determine the second microstructure quality of the polycrystalline structure for each leached component, the second microstructure quality of the polycrystalline structure being less porous when the second data scattering range is less in comparison to the second data scattering ranges of other leached components.

Clause 15. The method of any of clauses 1 to 14, wherein the leached layer has at least a portion of a by-product material removed from therein.

Clause 16. The method of any of clauses 1 to 15, further comprising mounting at least a portion of the leached components to a tool based upon the overall quality of the polycrystalline structure.

Clause 17. A method of characterizing a quality of a polycrystalline structure, comprising: obtaining a leached component comprising a polycrystalline structure, the polycrystalline structure comprising a leached layer and an unleached layer positioned adjacent to the leached layer, the leached layer having at least a portion of a catalyst material removed from therein; measuring at least one measured capacitance value of the leached component; measuring at least one measured eddy current value of the leached component; characterizing a quality of the polycrystalline structure based upon the at least one measured capacitance value of the leached component and upon the at least one measured eddy current value of the leached component, the quality comprising at least one of an estimated leaching depth of the leached component, a relative amount of catalyst remaining within the leached layer, and a relative porosity of the polycrystalline structure of the leached component.

Clause 18. The method of clause 17, wherein the leached layer has at least a portion of a by-product material removed from therein.

Clause 19. The method of clause 17 or clause 18, further comprising cleaning the polycrystalline structure from one or more by-product materials, wherein the by-product materials were deposited within the polycrystalline structure during a leaching process that removes at least a portion of the catalyst material from therein and forms the leached component.

Clause 20. The method of any of clauses 17 to 19, further comprising depolarizing the leached component, the depolarizing being performed in at least one of before measuring each measured capacitance value and after measuring each measured capacitance value and in at least one of before measuring each measured eddy current value and after measuring each measured eddy current value.

Clause 21. The method of any of clauses 17 to 20, further comprising mounting at least a portion of the leached components to a tool based upon the overall quality of the polycrystalline structure.

Clause 22. The method of any of clauses 17 to 21, wherein the one or more measured eddy current value comprises at least one of an impedance amplitude and a phase angle shift.

## Claims

1. A method of characterizing a quality of a polycrystalline structure, comprising:

   obtaining a leached component comprising a polycrystalline structure, the polycrystalline structure comprising a leached layer and an unleached layer positioned adjacent to the leached layer, the leached layer having at least a portion of a catalyst material removed from therein;
   measuring at least one measured capacitance value of the leached component;
   measuring at least one measured eddy current value of the leached component;
   characterizing a first quality of the polycrystalline structure, the first quality being based upon the at least one measured capacitance value;
   characterizing a second quality of the polycrystalline structure, the second quality being based upon the at least one measured eddy current value; and
   determining an overall quality of the polycrystalline structure, the overall quality being based upon the first quality and the second quality.

2. The method of Claim 1, further comprising:

   obtaining a first calibration curve showing a relationship between a plurality of capacitance values and a plurality of actual leaching depth within the polycrystalline structure; and
   obtaining a second calibration curve showing a relationship between a plurality of eddy current values and the plurality of actual leaching depth within the polycrystalline structure;
   wherein characterizing the first quality of the polycrystalline structure comprises using the one or more measured capacitance values of the leached component and the first calibration curve to estimate a first actual leaching depth within the leached component, and

wherein characterizing the second quality of the polycrystalline structure comprises using the one or more measured eddy current values of the leached component and the second calibration curve to estimate a second actual leaching depth within the leached component.

3. The method of Claim 2, wherein the overall quality comprises averaging the first actual leaching depth and the second actual leaching depth.

4. The method of any of Claims 1 to 3, wherein the one or more measured eddy current value comprises at least one of an impedance amplitude and a phase angle shift.

5. The method of any of Claims 1 to 4, further comprising:

obtaining a first calibration curve showing a relationship between a plurality of capacitance values and a plurality of actual leaching depth within the polycrystalline structure; and
obtaining a second calibration curve showing a relationship between a plurality of eddy current values and the plurality of actual leaching depth within the polycrystalline structure,
wherein characterizing the first quality of the polycrystalline structure comprises:

determining a first average of the at least one measured capacitance value of the leached component; and
using the first average and the first calibration curve to estimate a first actual leaching depth within the leached component, and

wherein characterizing the second quality of the polycrystalline structure comprises:

determining a second average of the at least one measured eddy current value of the leached component; and
using the second average and the second calibration curve to estimate a second actual leaching depth within the leached component.

6. The method of Claim 5, wherein the overall quality comprises averaging the first actual leaching depth and the second actual leaching depth.

7. The method of Claim 5, wherein the one or more measured eddy current value comprises at least one of an impedance amplitude and a phase angle shift.

8. The method of any of Claims 1 to 7, further comprising depolarizing the leached component.

9. The method of Claim 8, wherein depolarizing the leached component is performed in at least one of before measuring each measured capacitance value and after measuring each measured capacitance value and in at least one of before measuring each measured eddy current value and after measuring each measured eddy current value.

10. The method of Claim 8 or Claim 9, wherein depolarizing the leached component comprises at least one of grounding the leached component, wrapping the leached component in a depolarizing material, heat treating the leached component, placing the leached component in a salt solution, and waiting a period of time.

11. The method of any of Claims 1 to 10, further comprising cleaning the polycrystalline structure from one or more by-product materials, wherein the by-product materials were deposited within the polycrystalline structure during a leaching process that removes at least a portion of the catalyst material from therein and forms the leached component.

12. The method of any of Claims 1 to 11, further comprising:

measuring a plurality of measured capacitance values for each of a plurality of the leached components, each leached component being formed during a same leaching process; and
measuring a plurality of measured eddy current values for each of the plurality of the leached components;
wherein characterizing the first quality of the polycrystalline structure comprises using the plurality of measured capacitance values for each leached component to determine a first microstructure quality of the polycrystalline structure for each leached component, and
wherein characterizing the second quality of the polycrystalline structure comprises using the plurality of measured eddy current values for each leached component to determine a second microstructure quality of the polycrystalline structure for each leached component.

13. The method of Claim 12, further comprising:

determining a first data scattering range for each of the plurality of the leached components from the plurality of measured capacitance values; and
determining a second data scattering range for each of the plurality of the leached components from the plurality of measured eddy current values,

wherein characterizing the first quality of the polycrystalline structure comprises using the first data scattering range to determine the first microstructure quality of the polycrystalline structure for each leached component, the first microstructure quality of the polycrystalline structure being less porous when the first data scattering range is less in comparison to the first data scattering ranges of other leached components, and

wherein characterizing the second quality of the polycrystalline structure comprises using the second data scattering range to determine the second microstructure quality of the polycrystalline structure for each leached component, the second microstructure quality of the polycrystalline structure being less porous when the second data scattering range is less in comparison to the second data scattering ranges of other leached components.

14. The method of any of Claims 1 to 13, wherein the leached layer has at least a portion of a by-product material removed from therein.

15. The method of any of Claims 1 to 14, further comprising mounting at least a portion of the leached components to a tool based upon the overall quality of the polycrystalline structure.

**FIG. 1**
**(Prior Art)**

**FIG. 2**
**(Prior Art)**

**FIG. 3**
**(Prior Art)**

**FIG. 4**

FIG. 5

*FIG. 6*

FIG. 7A

FIG. 7B

LEFT CURSOR: 1:09 MIN
RIGHT CURSOR: 3.4 S
SELECTION: 1:06 MIN    530 KB

FIG. 7C

FIG. 8

**FIG. 9**

*1000*

**FIG. 10A**

*1000*

**FIG. 10B**

1100

1110 — START

1120 — OBTAIN ONE OR MORE LEACHED COMPONENTS FROM A SAME LEACHED BATCH, THE LEACHED COMPONENT INCLUDING A POLYCRYSTALLINE STRUCTURE HAVING A PLURALITY OF CATALYST MATERIAL THEREIN, THE POLYCRYSTALLINE STRUCTURE HAVING A LEACHED LAYER AND A NON-LEACHED LAYER BEING POSITIONED ADJACENTLY BELOW THE LEACHED LAYER, WHEREIN AT LEAST A PORTION OF THE CATALYST MATERIAL HAS BEEN REMOVED FROM THE LEACHED LAYER

1130 — MEASURE A PLURALITY OF CAPACITANCE VALUES OF EACH OF THE LEACHED COMPONENTS

1140 — MEASURE A PLURALITY OF EDDY CURRENT VALUES OF EACH OF THE LEACHED COMPONENTS

1150 — DETERMINE A FIRST AMOUNT OF DATA SCATTERING BASED UPON THE PLURALITY OF CAPACITANCE VALUES FOR EACH OF THE LEACHED COMPONENTS

1160 — DETERMINE A SECOND AMOUNT OF DATA SCATTERING BASED UPON THE PLURALITY OF EDDY CURRENT VALUES FOR EACH OF THE LEACHED COMPONENTS

1170 — DETERMINE A FIRST QUALITY OF THE LEACHED COMPONENT BASED UPON THE FIRST AMOUNT OF DATA SCATTERING

1180 — DETERMINE A SECOND QUALITY OF THE LEACHED COMPONENT BASED UPON THE SECOND AMOUNT OF DATA SCATTERING

1190 — DETERMINE AN OVERALL QUALITY OF THE LEACHED COMPONENT BASED UPON THE FIRST QUALITY AND THE SECOND QUALITY

1195 — END

**FIG. 11**

*FIG. 12*

**EP 2 631 637 A2**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 40118812 A
- US 40123112 A
- US 40145212 A